# FASCICULE DE BREVET EUROPEEN

(11) **EP 3 402 539 B1**
(45) Date de publication et mention de la délivrance du brevet: **10.12.2025**
(21) Numéro de dépôt: 17706532.3
(22) Date de dépôt: 16.01.2017
(51) Int. Cl.: A61L 2/20, A61L 2/24, B01D 46/00

(54) **PROCEDE POUR LA STERILISATION DE MOYENS DE FILTRATION DE GAZ, NOTAMMENT D'AIR DE SOUFFLAGE**
VERFAHREN ZUR STERILISIERUNG VON GASFILTERMITTELN, INSBESONDERE LUFTBLASEN
PROCESS FOR STERILIZING GAS FILTRATION MEANS, IN PARTICULAR BLOWING AIR

(30) Priorité: 15.01.2016 FR 1650326
(43) Date de publication de la demande: 21.11.2018
(73) Titulaire: Sidel Participations, 76930 Octeville-sur-Mer (FR)
(72) Inventeur: QUETEL, François, 76930 Octeville-sur-mer (FR); YGER, Benjamin, 76930 Octeville-sur-mer (FR); LETELLIER, Sandy, 76930 Octeville-sur-mer (FR); MARIE, Jeremy, 76930 Octeville-sur-mer (FR)
(74) Mandataire: Sidel Group
(86) Numéro de dépôt international: PCT/FR2017/050087
(87) Numéro de publication internationale: WO 2017/121973

(56) Documents cités:
- EP-A1- 1 283 061
- EP-A1- 3 034 151
- EP-A2- 0 243 073
- EP-A2- 0 815 919
- WO-A1-93/17726
- US-A1- 2009 169 421
- US-A1- 2012 020 848

## Description

La présente invention concerne un procédé pour la stérilisation de moyens de filtration de gaz, notamment d'air de soufflage.

La présente invention concerne plus particulièrement un procédé de stérilisation de moyens de filtration de gaz pour la stérilisation de moyens de filtration de gaz selon ledit procédé de stérilisation.

On connait de l'état de la technique de nombreux types de moyens de filtration de gaz et tout particulièrement de filtration d'air.

De tels moyens de filtration de gaz sont notamment utilisés pour la filtration d'air de soufflage dans des installations de fabrication de récipients en matière thermoplastique, la fabrication de récipients en matière thermoplastique étant obtenue par soufflage (ou par étirage-soufflage) dans un moule de préformes chaudes au moyen d'au moins un fluide sous pression.

Les récipients, tels que des bouteilles, flacons ou pots, sont généralement obtenus à partir de préformes qui sont préalablement fabriquées par moulage par injection de matière thermoplastique, telle que du PET (PolyEthylène Terephtalate), et qui sont ultérieurement conditionnées thermiquement pour obtenir des préformes chaudes aptes au moulage.

Pour ce faire, une installation comporte au moins un four pour le conditionnement thermique des préformes associé à une machine de moulage (ou « souffleuse ») pour le moulage des récipients à partir desdites préformes chaudes. L'installation comporte encore avantageusement une unité de stérilisation pour stériliser au moins l'intérieur des préformes de manière à obtenir des récipients dits « stériles ».

Pour réaliser le moulage par soufflage ou par étirage-soufflage d'une préforme chaude, il est connu d'utiliser pour le soufflage au moins un fluide, généralement un gaz, tel que de l'air comprimé à des pressions pouvant atteindre des valeurs de 30 ou 40 bars selon les applications.

L'air utilisé pour le soufflage est introduit, par l'ouverture (le col) de la préforme chaude placée dans un moule de sorte que l'air de soufflage entre directement en contact avec la surface interne du récipient.

Or, la surface interne est elle-même destinée à être ultérieurement en contact avec le contenu du récipient.

La qualité de l'air de soufflage, plus particulièrement l'absence de contaminants, tels que des micro-organismes, des particules comme des poussières, etc., est donc un paramètre important à prendre en compte afin de pouvoir garantir dans le cas de conditionnement agro-alimentaire une bonne conservation du produit conditionné, notamment une durée de conservation, et la sécurité du consommateur.

Or, la qualité de l'air comprimé utilisé pour le soufflage est déterminée par un ensemble de facteurs, depuis la qualité de l'air atmosphérique aspiré qui varie selon l'environnement du site industriel et sa localisation par rapport à des sources polluantes, jusqu'à l'état du réseau de distribution et/ou de l'installation.

L'air atmosphérique aspiré et comprimé par au moins un compresseur présente par exemple un degré d'hygrométrie plus ou moins important or l'humidité favorise la corrosion et le développement des micro-organismes.

Une attention particulière est également portée dans le choix des compresseurs qui, en raison notamment de la conception de leurs moyens de lubrification, sont susceptibles d'engendrer une contamination chimique de l'air par exemple par de l'huile de lubrification ou encore des poussières de téflon.

C'est la raison pour laquelle, l'air comprimé destiné à être utilisé pour le soufflage est préalablement traité et tout particulièrement filtré par des moyens de filtration de gaz afin d'obtenir un air de soufflage qui soit « stérile », c'est-à-dire notamment exempt de micro-organismes.

L'air comprimé utilisé pour le soufflage est généralement successivement filtré par un système de filtration comportant différents moyens de filtration de gaz.

De manière non limitative, un tel système de filtration d'air destiné au soufflage comporte par exemple des moyens de filtration multiples qui, agencés en série, sont destinés à délivrer en sortie un air stérile.

L'air y est par exemple successivement filtré par des premiers moyens de filtration de type « FFP » pour obtenir notamment un déshuilage, une épuration en eau et une élimination des poussières, puis des deuxièmes moyens de filtration de type « AK » (à charbons actifs) pour ôter toutes les vapeurs d'huile et d'hydrocarbures gazeux pouvant en outre entraîner une nuisance olfactive et gustative, et enfin par des troisièmes moyens de filtration de type « SRF » pour retenir les micro-organismes.

En effet, l'air de soufflage est susceptible d'être un vecteur de contamination de l'intérieur de la préforme, et donc du récipient, en y introduisant des contaminants et tout particulièrement des micro-organismes (virus, germes, spores, etc.).

La maîtrise de la qualité de l'air comprimé utilisé pour le soufflage est encore plus importante lorsque, dans le procédé de fabrication des récipients, le remplissage des récipients est réalisé dans un environnement aseptique directement après le moulage du récipient obtenu par soufflage ou par étirage-soufflage d'une préforme chaude.

En effet, dans un tel procédé de fabrication, la stérilisation est généralement effectuée en amont sur les préformes, avant leur transformation en récipients, de sorte qu'il est ensuite essentiel de prévenir les risques de contamination des préformes stérilisées, comme celle des récipients fabriqués à partir de ces préformes.

L'invention vise à stériliser des moyens de filtration de gaz, notamment mais non exclusivement des moyens de filtration d'air de soufflage, tels que par exemple les moyens de filtration de type « SRF » qui sont utilisés pour éliminer les micro-organismes de l'air comprimé destiné au soufflage pour la fabrication de récipients stériles.

On recherche des solutions pour stériliser de tels moyens de filtration de gaz, avant une première utilisation comme après une certaine utilisation.

La stérilisation des moyens de filtration doit permettre de détruire les micro-organismes présents, résultant notamment de l'utilisation desdits moyens de filtration, afin d'en prévenir le développement dans les moyens de filtration et ainsi éliminer les risques de migration de tels micro-organismes en aval desdits moyens de filtration en cours d'utilisation.

Dans le cas d'une application industrielle comme celle de la fabrication de récipients destinés au conditionnement de produits agro-alimentaires, il est important de pouvoir garantir une qualité et tout particulièrement la stérilité de l'air de soufflage filtré grâce à de tels moyens de filtration de gaz.

Avec une telle stérilisation, on recherche pour des raisons économiques à réduire également la fréquence de changement des moyens de filtration.

Pour stériliser des moyens de filtration de gaz (ou filtres), il est connu de l'état de la technique d'utiliser de la vapeur d'eau, notamment pour stériliser des filtres destinés à filtrer de l'air à basse pression.

Une telle stérilisation à la vapeur d'eau a cependant également été utilisée pour stériliser des moyens de filtration de gaz destinés à la filtration d'air de soufflage, tels que des filtres prévus pour fonctionner à des pressions élevées comme les filtres de type FFP précités.

La stérilisation est alors généralement réalisée avec une vapeur d'eau de qualité alimentaire, c'est-à-dire une vapeur caractérisée par une teneur en eau très basse et l'absence d'impuretés.

Une telle stérilisation à la vapeur d'eau des moyens de filtration utilisés pour filtrer l'air de soufflage dans une installation de fabrication de récipients en matière thermoplastique n'a toutefois pas donné satisfaction pour les raisons détaillées ci-après.

Tout d'abord, on a pu constater que la qualité de la vapeur d'eau utilisée lors de la stérilisation était disparate. Généralement d'une qualité insuffisante, parfois médiocre, la qualité de la vapeur d'eau s'avère surtout hétérogène d'un site industriel à un autre pour une même installation de fabrication de récipients.

Ensuite et même avec de la vapeur d'eau de qualité, la stérilisation des moyens de filtration obtenue n'est pas satisfaisante.

Dans ces conditions et pour préserver la qualité de filtration, il n'est ainsi pas rare de constater une augmentation de la fréquence de stérilisation des moyens de filtration, et également une réduction sensible de leur durée de vie.

Or, la stérilisation des moyens de filtration (comme leur changement) impose d'en interrompre l'utilisation, soit dans notre exemple d'utilisation un arrêt de la fabrication de récipients qui est particulièrement préjudiciable économiquement.

Des études ont permis de constater la présence d'un excès d'eau dans les moyens de filtration après de telles opérations de stérilisation par vapeur.

On a également pu établir que les températures induites par l'utilisation de la vapeur d'eau, supérieures à 120°C, causaient des dommages aux moyens de filtration et ce faisant en dégradaient les propriétés de filtration pour un usage ultérieur.

Outre l'absence d'homogénéité dans la qualité de la vapeur d'eau utilisée pour la stérilisation, on a notamment pu constater qu'une accumulation de condensats d'eau se produisait dans les moyens de filtration ainsi stérilisés, ces condensats restant emprisonnés dans les moyens de filtration et se révélant particulièrement difficiles à éliminer.

Dans une stérilisation à la vapeur d'eau, on utilise de l'air comprimé à température ambiante pour éliminer l'eau afin de sécher les moyens de filtration. Un tel séchage nécessite une importante quantité d'air dès lors que les débits mis en œuvre sont par exemple de l'ordre de 2000 à 3000 m³/h.

La pression de l'air utilisé pour le séchage ne doit pas excéder certaines valeurs de pression, par exemple 4 bars, sous peine de provoquer des détériorations de la structure des moyens de filtration.

En effet, la présence d'eau dans les moyens de filtration s'oppose au passage de l'air de séchage à travers les moyens de filtration et, en les colmatant, l'eau en modifie la perte de charge.

Enfin, l'air de séchage tend à pousser en force les condensats à travers les moyens de filtration et ceci alors que les moyens de filtration de gaz sont hydrophobes.

Par conséquent, la stérilisation à la vapeur d'eau provoque une dégradation des moyens de filtration pouvant aller jusqu'à leur destruction, destruction qui présente par ailleurs un caractère totalement imprévisible.

Il résulte de ce qui précède que les performances des moyens de filtration de l'air de soufflage ainsi stérilisés à la vapeur d'eau se dégradent et que leur durée de vie s'en trouve considérablement réduite.

De plus, une élimination imparfaite des condensats d'eau après une stérilisation à la vapeur d'eau des moyens de filtration peut s'avérer particulièrement préjudiciable ensuite lors de la reprise de leur utilisation.

En effet, les condensats d'eau encore présents dans les moyens de filtration (du fait de l'excès d'eau et de la difficulté de son élimination) vont alors se retrouver poussés vers l'aval par le gaz tel que l'air de soufflage.

Dans le cas d'une installation de fabrication de récipients, l'air de soufflage sous haute pression poussent les résidus d'eau en aval des moyens de filtration, c'est à dire dans le circuit de soufflage puis dans les récipients fabriqués eux-mêmes, avec pour conséquence de mouiller le circuit de soufflage et surtout d'introduire de l'humidité à l'intérieur des récipients fabriqués.

Or, la présence d'humidité à l'intérieur des récipients fabriqués accroît les risques de contamination microbiologiques.

Par ailleurs, il existe un autre mode opératoire pour stériliser les filtres que l'utilisation de la vapeur comme décrit précédemment, il s'agit de d'une stérilisation par voie chimique, c'est-à-dire notamment l'usage de peroxyde d'hydrogène pour stériliser les filtres.

Les documents EP0815919, US2009/169421, EP0243073 décrivent la stérilisation par voie chimique en imprégnant en une seule fois des filtres de peroxyde d'hydrogène soit à l'état liquide ou soit à l'état gazeux, et en l'activant le peroxyde d'hydrogène par de l'air chaud. On connaît aussi les documents US 2012/020848 A1, EP 1 283 061 A1, WO 93/17726 A1.

Le but de la présente invention est notamment de résoudre les inconvénients précités et tout particulièrement de proposer une nouvelle solution pour stériliser des moyens de filtration de gaz, tels que les moyens de filtration de l'air de soufflage utilisés dans une installation de fabrication de récipients en matière thermoplastique.

Dans ce but, l'invention propose un procédé de stérilisation de moyens de filtration de gaz selon la revendication 1.

Avantageusement, le procédé de stérilisation selon l'invention permet de stériliser efficacement les moyens de filtration de gaz, tout en préservant leur intégrité de manière à en maintenir les performances de filtration et en augmenter la durée de vie, à tout le moins la préserver.

En effet, les risques de dégradation, voire de destruction, de la structure des moyens de filtration sont supprimés avec une stérilisation chimique selon l'invention.

Par comparaison avec la stérilisation par vapeur d'eau, l'invention ne nécessite pas une importante quantité d'air car les condensats de peroxyde d'hydrogène sont successivement éliminés par chauffage au moyen d'air chaud.

La stérilisation selon l'invention est plus économique en air avec un débit d'air chaud qui est plus de cent fois inférieur à celui de l'air utilisé pour le séchage dans le procédé de stérilisation à la vapeur d'eau ou pour l'évaporation du peroxyde d'hydrogène injecté continu selon l'état de la technique.

Avantageusement, le mélange stérilisant comporte une dose déterminée de peroxyde d'hydrogène à l'état de vapeur qui se dépose par condensation sur les moyens de filtration lorsque le mélange gazeux d'air chaud et de vapeur entre en contact avec les moyens de filtration.

Avantageusement, les condensats de peroxyde d'hydrogène sont ensuite éliminés progressivement par évaporation grâce au chauffage avec de l'air chaud, ladite évaporation étant réalisée au moins entre l'injection de deux doses successives de peroxyde d'hydrogène liquide.

Selon une caractéristique importante, il doit être bien compris que le procédé de stérilisation selon l'invention est un procédé de nature « chimique ».

En effet et par comparaison avec la stérilisation par vapeur d'eau, l'effet stérilisant ne résulte pas (ou pas uniquement) d'une destruction thermique des micro-organismes présents dans les moyens de filtration, par l'apport de chaleur dû à la vapeur d'eau.

Dans l'invention, l'air chaud a une double fonction, celle d'activer thermiquement le peroxyde d'hydrogène condensé sur les moyens de filtration et celle d'en éliminer selon la durée d'application de l'air chaud au moins une partie par évaporation.

L'air chaud va d'abord provoquer une élimination progressive par évaporation de l'eau présente dans les condensats de peroxyde d'hydrogène, ce qui va avoir pour effet d'en augmenter progressivement la concentration et donc en accroître l'effet stérilisant.

Dans le cas d'un agent stérilisant formé par du peroxyde d'hydrogène (H₂O₂), on désigne par activation thermique le fait que l'air chaud agit sur le peroxyde d'hydrogène pour en casser les liaisons chimiques ce qui provoque l'apparition de radicaux libres (OH) actifs qui vont détruire les micro-organismes et permettre d'obtenir le degré de stérilisation recherché.

L'agent stérilisant utilisé pour la stérilisation des moyens de filtration selon l'invention est avantageusement du peroxyde d'hydrogène (H₂O₂), connu pour ses propriétés germicides, notamment dans le domaine agro-alimentaire.

Lorsque le peroxyde d'hydrogène est également utilisé dans l'installation de fabrication de récipients pour stériliser au moins l'intérieur des préformes en matière thermoplastique, la source d'alimentation en peroxyde d'hydrogène est par exemple susceptible d'être une source commune aux dispositifs de stérilisation des moyens de filtration de l'air de soufflage, d'une part, et des préformes, d'autre part.

Avantageusement, le procédé de stérilisation des moyens de filtration selon l'invention comporte tour à tour, c'est-à-dire en alternance, au moins une étape d'application du mélange gazeux comportant la vapeur de peroxyde d'hydrogène obtenue à partir de ladite dose déterminée et une étape de stérilisation utilisant de l'air chaud.

Lesdites étapes d'application et de stérilisation constituent respectivement une séquence qui est avantageusement répétées un nombre « n » de fois pour réaliser un cycle de stérilisation, l'évaporation des condensats de peroxyde d'hydrogène étant réalisée au moins dans l'intervalle de temps donné entre deux injections successives d'une dose déterminée de peroxyde d'hydrogène.

Après la répétition « n » fois de ladite séquence le procédé de stérilisation comporte avantageusement en fin de cycle, une étape de stérilisation complémentaire consistant, comme lors dudit intervalle de temps donné, à faire circuler uniquement de l'air chaud à travers lesdits moyens de filtration pour achever au besoin l'élimination du peroxyde d'hydrogène par évaporation.

L'étape de stérilisation complémentaire est réalisée pendant une durée qui est notamment déterminée en fonction de la valeur dudit intervalle de temps, ladite étape pouvant être supprimée lorsque ledit intervalle de temps est suffisant pour garantir une évaporation totale des condensats de peroxyde d'hydrogène déposé après l'injection de chaque dose déterminée.

L'injection séquentielle du peroxyde d'hydrogène, c'est-à-dire la répétition alternée desdites étapes d'application d'une fraction de la quantité déterminée de vapeur puis d'air chaud pour la stérilisation permet avantageusement de ne pas saturer les moyens de filtration, évitant notamment tout colmatage.

Grâce à l'injection séquentielle d'une dose donnée de peroxyde d'hydrogène, on dépose une moindre quantité de peroxyde d'hydrogène qui peut ensuite être totalement ou au moins partiellement éliminée par l'air chaud pendant ledit intervalle de temps donné et cela avant de procéder à une nouvelle application résultant de l'injection d'une nouvelle dose donnée.

Avantageusement, l'injection séquentielle permet de réduire la quantité d'énergie nécessaire pour stériliser et de préserver les moyens de filtration des dégradations notamment consécutives aux effets de la température.

La quantité totale d'air chaud (et donc d'énergie) nécessaire à l'évaporation de la totalité du peroxyde d'hydrogène est moindre lorsque ledit peroxyde d'hydrogène est appliqué de manière discontinue que si la quantité correspondant à la somme des doses était appliquée en une seule fois.

Avantageusement, la température de l'air chaud requise pour évaporer l'équivalent d'une dose déterminée est également moindre de sorte que cela participe à préserver les moyens de filtration des dégradations telles que celles auparavant observées par comparaison avec la vapeur d'eau.

Après son évaporation, le peroxyde d'hydrogène à l'état gazeux est libre de traverser complètement les moyens de filtration, de pénétrer en profondeur à l'intérieur mais sans jamais en altérer la structure comme le faisait les condensats de vapeur d'eau.

Pour une quantité égale de vapeur de peroxyde d'hydrogène, la stérilisation obtenue des moyens de filtration est meilleure en effectuant successivement « n » fois la séquence desdites étapes d'application et de stérilisation qu'en projetant en une seule fois une quantité équivalente de vapeur de peroxyde d'hydrogène.

Selon d'autres caractéristiques du procédé de stérilisation
- le procédé comporte au moins une étape de vaporisation consistant à vaporiser ladite dose donnée de peroxyde d'hydrogène à l'état liquide dans des moyens d'évaporation pour obtenir ledit mélange gazeux utilisé lors de l'étape d'application ;
- le procédé comporte au moins une étape d'injection consistant à injecter séquentiellement avec ledit intervalle de temps donné entre deux injections successives ladite dose donnée de peroxyde d'hydrogène à l'état liquide dans un flux continu d'air chaud et à introduire l'ensemble dans lesdits moyens d'évaporation pour obtenir ledit mélange gazeux ;
- l'étape d'injection de ladite dose donnée de peroxyde d'hydrogène est réalisée séquentiellement en commandant sélectivement des moyens de régulation, respectivement en position d'ouverture pendant un laps de temps donné et en position de fermeture pendant un intervalle de temps donné entre deux injections successives de ladite dose donnée de peroxyde d'hydrogène à l'état liquide ;
- le procédé de stérilisation consiste à réaliser au moins un cycle au cours duquel une séquence, comportant lesdites étapes d'application et de stérilisation, est répétée un nombre « n » de fois ;
- le procédé comporte au moins une étape de stérilisation complémentaire consistant à faire circuler uniquement de l'air chaud à travers lesdits moyens de filtration pendant une durée qui est déterminée en fonction de l'intervalle de temps pour garantir une évaporation du peroxyde d'hydrogène.

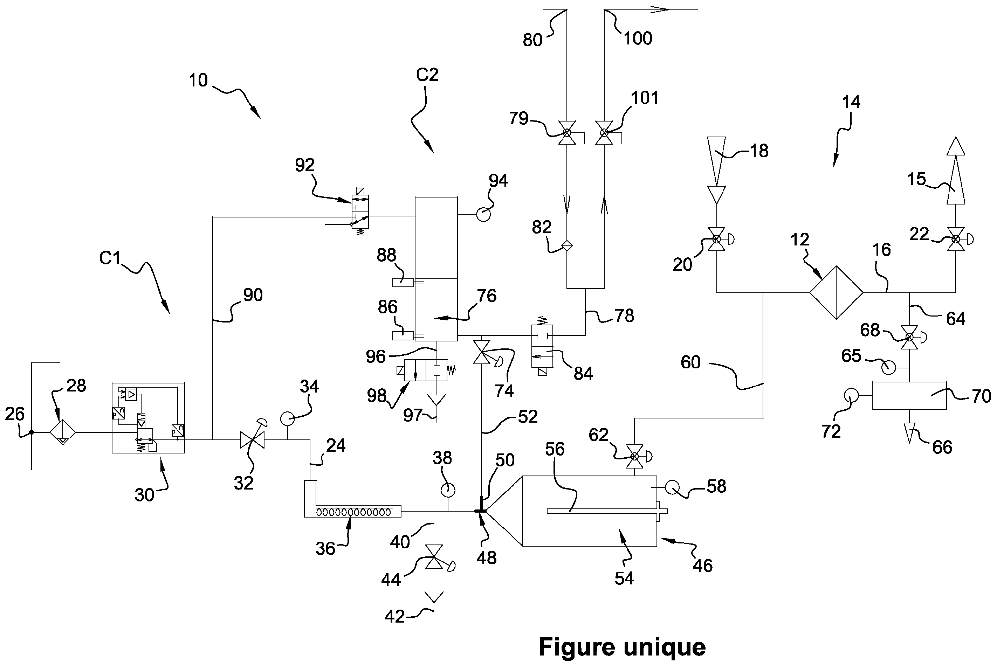

D'autres caractéristiques et avantages apparaîtront à la lecture de la description qui va suivre pour la compréhension de laquelle on se reportera à la figure unique qui représente schématiquement un exemple de réalisation d'un dispositif de stérilisation destiné à être intégré à une installation de fabrication de récipients pour en stériliser les moyens de filtration de gaz.

On a représenté sur la figure un schéma pneumatique illustrant un exemple de réalisation d'un dispositif 10 de stérilisation pour stériliser des moyens 12 de filtration de gaz.

Dans l'exemple de réalisation, lesdits moyens 12 de filtration de gaz sont constitués par des moyens de filtration d'air de soufflage.

Les moyens 12 de filtration sont destinés à filtrer de l'air comprimé à haute pression pour délivrer un air stérile apte à être utilisé comme fluide de soufflage dans une installation (non représentée) de fabrication de récipients en matière thermoplastique à partir de préformes chaudes.

L'air comprimé à haute pression utilisé pour le soufflage des préformes chaudes en récipients, ci-après désigné l'air de soufflage, doit être un air « stérile », c'est-à-dire exempt de contaminants et tout particulièrement de micro-organismes.

Par « haute pression », on désigne d'une manière générale aussi bien la pression finale de l'air de soufflage, qui peut atteindre les 25 ou 40 bars pour certaines applications, qu'une pression inférieure destinée à un pré-soufflage ou également à un soufflage, et par exemple comprise entre 7 et 20 bars.

Selon l'invention, on procède à une stérilisation chimique des moyens 12 de filtration d'air en utilisant au moins un agent stérilisant à l'état de vapeur qui, après avoir été déposé sur lesdits moyens 12 de filtration, est activé thermiquement et éliminé par évaporation au moyen d'un air chaud produit par le dispositif 10 de stérilisation.

L'agent stérilisant utilisé pour la stérilisation des moyens de filtration est du peroxyde d'hydrogène (H₂O₂).

Dans l'exemple de réalisation illustré sur la figure, les moyens 12 de filtration comportent au moins un filtre.

En variante non représentée, les moyens 12 de filtration comportent plus d'un filtre pour filtrer l'air destiné au soufflage.

Pour délivrer de l'air stérile, les moyens 12 de filtration sont par exemple constitués par au moins un filtre de type « SRF ».

De préférence, lesdits moyens 12 de filtration représentés sur la figure forment les derniers moyens de filtration d'un système de filtration multiples du type décrit précédemment et sont notamment aptes à réaliser une filtration finale de l'air avant son utilisation pour le soufflage.

De préférence, ledit système de filtration multiple comporte des moyens de déshumidification de l'air.

De tels moyens 12 de filtration sont plus particulièrement destinés à éliminer les micro-organismes de l'air et peuvent, pour ce faire, comporter plus d'un étage de filtration.

En variante non représentée, les moyens 12 de filtration comportent au moins deux étages de filtration, par exemple en série, pour réaliser une double filtration. Indépendamment du nombre d'étages de filtration, un étage de filtration peut également comporter plus d'un moyen de filtration (ou filtre), par exemple deux filtres en parallèle.

On a représenté sur la figure une partie seulement d'un circuit 14 de soufflage d'une machine de moulage ou « souffleuse » (non représentée).

De manière connue, une telle machine de moulage est par exemple de type rotative et est pourvue d'une pluralité de postes pour le formage par soufflage ou par étirage-soufflage de récipients en matière thermoplastique à partir de préformes chaudes.

Les moyens 12 de filtration sont agencés dans une conduite 16 principale. La conduite 16 est raccordée à une extrémité, en amont des moyens 12 de filtration, à une source 18 d'air de soufflage délivrant ledit air comprimé sous pression et dont la valeur maximale est déterminée en fonction des applications.

Des moyens 20 de régulation sont disposés dans la conduite 16 entre ladite source 18 d'air de soufflage et les moyens 12 de filtration, pour pouvoir notamment isoler lesdits moyens 12 de filtration lors de la stérilisation au moyen dudit dispositif 10 de stérilisation.

En position d'ouverture, les moyens 20 de régulation autorisent, depuis la source 18 d'air de soufflage située en amont, une circulation d'air de l'amont vers l'aval dans la conduite 16 et, en position de fermeture, les moyens 20 de régulation interrompent ladite circulation d'air dans la conduite 16, entre la source 18 d'air de soufflage et les moyens 12 de filtration situés en aval.

La conduite 16 est raccordée à l'autre extrémité, en aval des moyens 12 de filtration, à au moins une partie 15 du circuit 14 de soufflage par laquelle l'air de soufflage filtré par lesdits moyens 12 de filtration est acheminé jusqu'à des moyens de soufflage (non représentés), tels qu'au moins une tuyère, associés à un moule de ladite machine de moulage de récipients.

Des moyens 22 de régulation sont disposés dans la conduite 16 entre ladite partie 15 du circuit 14 de soufflage et les moyens 12 de filtration, notamment pour pouvoir isoler lesdits moyens 12 de filtration lors de la stérilisation et par rapport à ladite partie 15 du circuit 14 de soufflage.

De préférence, les moyens 20 et 22 de régulation sont formés par au moins une vanne telle qu'une électrovanne.

Les moyens 20 et 22 de régulation sont commandés sélectivement par une unité de contrôle (non représentée) entre au moins une position d'ouverture et une position de fermeture.

En position d'ouverture, les moyens 22 de régulation autorisent une circulation d'air de l'amont vers l'aval dans la conduite 16 et, en position de fermeture, les moyens 22 de régulation interrompent ladite circulation d'air dans la conduite 16, entre les moyens 12 de filtration et la partie 15 du circuit 14 de soufflage située en aval.

Avantageusement, le dispositif 10 de stérilisation est commandé sélectivement entre un état de veille dans lequel ledit dispositif 10 de stérilisation est inactif et un état d'utilisation dans lequel le dispositif 10 de stérilisation est actif, utilisé pour stériliser les moyens 12 de filtration.

De préférence et selon l'exemple de réalisation, le dispositif 10 de stérilisation équipe une installation de fabrication de récipients en matière thermoplastique par soufflage ou par étirage-soufflage de préformes chaudes.

Avantageusement, le dispositif 10 de stérilisation peut équiper une installation de fabrication, que ladite installation soit nouvelle ou existante.

En variante, le dispositif 10 de stérilisation constitue une unité indépendante de l'installation susceptible de stériliser différents moyens de filtration de gaz.

Le dispositif 10 de stérilisation est plus particulièrement associé à la machine de soufflage (ou souffleuse) d'une telle installation pour en stériliser les moyens 12 de filtration d'air.

Le dispositif 10 de stérilisation des moyens de filtration d'air selon l'invention est notamment susceptible d'y remplacer un dispositif de stérilisation à vapeur d'eau selon l'état de la technique et tel que décrit en préambule.

De préférence, le dispositif 10 de stérilisation est apte à stériliser des moyens 12 de filtration d'air de soufflage en place, c'est-à-dire dans la position d'utilisation que lesdits moyens 12 de filtration occupent lorsque des récipients sont fabriqués par l'installation et que le dispositif 10 de stérilisation inactif est à l'état de veille.

Avantageusement, aucune intervention n'est requise sur les moyens 12 de filtration, notamment pas d'intervention humaine pour procéder à un démontage et remontage.

La stérilisation est par conséquent susceptible d'être réalisée automatiquement en commandant le dispositif 10 de stérilisation.

Pour réaliser une telle stérilisation des moyens 12 de filtration d'air de soufflage au moyen du dispositif 10 de stérilisation, la fabrication de récipients doit être préalablement interrompue.

On procède initialement à un changement de mode de fonctionnement de l'installation en passant d'un mode de fonctionnement de fabrication de récipients dans lequel des récipients sont susceptibles d'être fabriqués à partir de préformes chaudes, à un autre mode de fonctionnement, dit d'intervention, dans lequel la fabrication de récipients cesse.

Des interventions telles que la stérilisation des moyens 12 de filtration d'air de soufflage au moyen du dispositif 10 de stérilisation sont alors notamment susceptibles d'être réalisées.

Le changement de mode de fonctionnement de l'installation en vue de procéder à une stérilisation des moyens 12 de filtration d'air de soufflage s'accompagne d'un changement d'état du dispositif 10 de stérilisation qui est activé, passant de l'état de veille à l'état d'utilisation.

On décrira ci-après le dispositif 10 de stérilisation de moyens de filtration selon l'exemple de réalisation illustré sur la figure et dont le fonctionnement sera décrit en détails ultérieurement.

Dans l'exemple de réalisation illustré sur la figure, le dispositif 10 de stérilisation comporte au moins un premier circuit C1 principalement pour l'air qui est associé à un deuxième circuit C2 pour l'agent stérilisant formé ici par du peroxyde d'hydrogène (H₂O₂).

Le dispositif 10 de stérilisation est notamment destiné à la mise en œuvre du procédé de stérilisation de moyens de filtration de gaz selon l'invention.

Le dispositif 10 de stérilisation est en particulier destiné à être utilisé pour la stérilisation de moyens de filtration d'air de soufflage dans une installation de fabrication de récipients en matière thermoplastique à partir de préformes chaudes.

Le premier circuit C1 du dispositif 10 de stérilisation comporte au moins une conduite 24 dont une extrémité amont est reliée à une source 26 d'alimentation en air comprimé.

De préférence, la source 26 d'alimentation en air comprimé est un réseau de distribution d'air comprimé délivrant de l'air à une pression déterminée, par exemple une basse pression d'environ 7 bars.

Avantageusement, le premier circuit C1 comporte des moyens 28 de filtration de l'air comprimé délivré par ladite source 26 d'alimentation.

De préférence, les moyens 28 de filtration permettent d'éliminer les contaminants présents dans l'air comprimé et sont agencés dans la conduite 24 en entrée du premier circuit C1, à l'extrémité amont raccordée à la source 26 d'alimentation.

Avantageusement, le premier circuit C1 comporte des moyens 30 de régulation de pression afin de réguler la pression de l'air comprimé circulant dans la conduite 24 pour assurer un débit constant.

Les moyens 30 de régulation de pression sont par exemple constitués par un régulateur de pression à membrane piloté pour réguler la pression dynamique de l'air comprimé.

De préférence, les moyens 30 de régulation de pression sont agencés dans la conduite 24, en aval des moyens 28 de filtration de l'air comprimé.

Avantageusement, le premier circuit C1 comporte des moyens 32 de régulation de la circulation d'air dans la conduite 24. De préférence, les moyens 32 de régulation sont formés par au moins une vanne telle qu'une électrovanne.

Les moyens 32 de régulation sont disposés dans la conduite 24, par exemple en aval des moyens 30 de régulation de pression et sont commandés pour établir sélectivement une circulation d'air comprimé dans la conduite 24.

Les moyens 32 de régulation sont commandés sélectivement par une unité de contrôle (non représentée) entre au moins une position d'ouverture et une position de fermeture.

De préférence, le dispositif 10 de stérilisation comporte une unité de contrôle pour commander notamment l'ensemble des moyens de régulation.

En position d'ouverture, les moyens 32 de régulation autorisent une circulation d'air comprimé de l'amont vers l'aval dans la conduite 24 et, en position de fermeture, les moyens 32 de régulation interrompent ladite circulation d'air dans la conduite 24.

De préférence, le premier circuit C1 comporte des moyens 34 de mesure de pression agencés pour mesurer la pression de l'air comprimé circulant dans ladite conduite 24.

Avantageusement, le premier circuit C1 comporte des moyens 36 de chauffage pour chauffer l'air comprimé mis sélectivement en circulation dans la conduite 24.

Les moyens 34 de mesure de pression sont de préférence agencés en aval des moyens 32 de régulation et en amont des moyens 36 de chauffage.

Les moyens 34 de mesure de pression sont par exemple formés par une sonde de pression, permettant notamment une surveillance de la pression dynamique de l'air en circulation dans la conduite 24 du premier circuit C1.

Les moyens 36 de chauffage comportent par exemple au moins un réchauffeur d'air qui est apte à chauffer l'air comprimé circulant dans la conduite 24 jusqu'à une température (Tc) de consigne donnée.

Les moyens 36 de chauffage de l'air sont commandés sélectivement pour que la température de l'air circulant vers l'aval soit au moins à ladite température (Tc) de consigne.

De préférence, la température (Tc) de consigne donnée est une température de l'ordre de 220°C. Une telle valeur de température (Tc) de consigne permet d'obtenir un air chaud présentant une température d'environ 110°C lorsqu'il entre en contact avec lesdits moyens 12 de filtration.

Une telle température de l'air chaud, avantageusement inférieure à 120°C, permet de ne pas détériorer les moyens 12 de filtration lorsque l'air chaud entre en contact avec eux.

Pour commander sélectivement les moyens 36 de chauffage et contrôler la température de l'air chaud, le circuit C1 du dispositif 10 de stérilisation comporte des moyens 38 de mesure de température.

Les moyens 38 de mesure de température sont aptes à mesurer la température de l'air chaud circulant dans la conduite 24 après avoir traversé les moyens 36 de chauffage formés par ledit au moins un réchauffeur.

De préférence, les moyens 38 de mesure de température de l'air sont disposés en aval desdits moyens 36 de chauffage.

Le circuit C1 du dispositif 10 de stérilisation comporte une conduite 40 de purge dont une extrémité amont est raccordée à la conduite 24 et dont l'autre extrémité aval est raccordée à des moyens 42 d'évacuation hors du dispositif 10 de stérilisation.

Les moyens 42 d'évacuation sont destinés à recueillir l'air, chaud ou non, notamment lorsque la température de l'air n'est pas conforme à ladite température (Tc) de consigne.

De préférence, la conduite 40 de purge est raccordée à la conduite 24 en aval des moyens 36 de chauffage de l'air.

Des moyens 44 de régulation sont disposés dans la conduite 40 de purge et sont commandés pour établir sélectivement une circulation d'air dans la conduite 40, en dérivation de la conduite 24.

De préférence, les moyens 44 de régulation sont formés par au moins une électrovanne.

Les moyens 44 de régulation sont commandés sélectivement par ladite unité de contrôle (non représentée) entre au moins une position d'ouverture et une position de fermeture.

En position d'ouverture, les moyens 44 de régulation autorisent une circulation d'air comprimé de l'amont vers l'aval dans la conduite 40 de purge et, en position de fermeture, les moyens 44 de régulation interrompent ladite circulation d'air dans la conduite 40 de purge.

La conduite 24 du premier circuit C1 comporte à une autre extrémité, en aval soit à l'opposé de la source 26 d'alimentation en air comprimé, au moins un évaporateur 46.

De préférence, un raccord 48 est interposé entre la conduite 24 et une entrée dudit au moins un évaporateur 46.

Le raccord 48, par exemple une forme en « T » inversé, comporte un conduit principal interne dans lequel débouchent des moyens 50 d'injection du peroxyde d'hydrogène.

Avantageusement, les moyens 50 d'injection de l'agent stérilisant formé par le peroxyde d'hydrogène sont aptes à le pulvériser sous la forme d'un brouillard formé de très fines gouttelettes.

Une conduite 52 d'injection du peroxyde d'hydrogène du deuxième circuit C2 du dispositif 10 de stérilisation est raccordée auxdits moyens 50 d'injection débouchant dans le conduit du raccord 48 pour injecter, dans l'air chaud le traversant, le peroxyde d'hydrogène à l'état liquide.

L'évaporateur 46 comporte une cavité 54 à l'intérieur de laquelle sont agencés au moins des moyens 56 de chauffage pour produire de la vapeur d'agent stérilisant à partir du mélange d'air chaud issu de la conduite 24 du premier circuit C1 et du peroxyde d'hydrogène à l'état liquide issu de la conduite 52 d'injection du deuxième circuit C2.

Des moyens 58 de mesure de température sont associés à l'évaporateur 46 pour mesurer la température du mélange présent à l'intérieur de la cavité 54 et constitué d'air chaud et de vapeur de peroxyde d'hydrogène, lequel est avantageusement introduit séquentiellement par doses au niveau du raccord 48.

L'évaporateur 46 est raccordé à la conduite 16 du circuit 14 de soufflage par une conduite 60. Une extrémité amont de la conduite 60 communique avec la cavité 54 de l'évaporateur 46 et l'autre extrémité aval de la conduite 60 se raccorde à la conduite 16.

La conduite 60 du dispositif 10 de stérilisation se raccorde à la conduite 16 entre les moyens 20 de régulation et les moyens 12 de filtration.

Des moyens 62 de régulation sont agencés dans la conduite 60 pour contrôler sélectivement la circulation dans ladite conduite 60 du mélange gazeux qui, constitué d'air chaud et de vapeur de peroxyde d'hydrogène produite par l'évaporateur 46, est destiné à être introduit dans le circuit 14 de soufflage et plus précisément dans la conduite 16 pour la stérilisation desdits moyens 12 de filtration.

De préférence, les moyens 62 de régulation sont formés par au moins une vanne telle qu'une électrovanne. Les moyens 62 de régulation sont commandés sélectivement par l'unité de contrôle (non représentée) entre au moins une position d'ouverture autorisant la circulation du mélange dans la conduite 60 et une position de fermeture interrompant la circulation dans la conduite 60.

Les moyens 58 de mesure de température sont notamment utilisés pour commander au moins les moyens 56 de chauffage et/ou les moyens 62 de régulation.

Une conduite 64 d'évacuation est prévue pour évacuer vers des moyens 66 de collecte, tels qu'un égout, des effluents du mélange après la stérilisation des moyens 12 de filtration.

La conduite 64 d'évacuation comporte une extrémité raccordée à la conduite 16 entre les moyens 12 de filtration et les moyens 22 de régulation, et une autre extrémité qui communique en aval avec lesdits moyens 66 de collecte.

Des moyens 68 de régulation, tels qu'une électrovanne, sont agencés dans la conduite 64 d'évacuation pour contrôler la circulation dans ladite conduite 64.

Les moyens 68 de régulation sont commandés sélectivement par l'unité de contrôle (non représentée) entre au moins une position d'ouverture autorisant la circulation dans la conduite 64 d'évacuation et une position de fermeture interrompant la circulation dans ladite conduite 64.

Une partie du mélange introduit dans la conduite 16 par l'intermédiaire de la conduite 60 est, après avoir stérilisé les moyens 12 de filtration, évacué par la conduite 64 d'évacuation vers les moyens 66 de collecte lorsque les moyens 68 de régulation sont en position d'ouverture.

Des moyens 65 de mesure de température sont disposés pour mesurer la température dans la conduite 64 d'évacuation.

De préférence, les moyens 65 de mesure de température sont constitués par un thermocouple.

Avantageusement, les premiers moyens 65 de mesure de température permettent de réaliser une surveillance de la température pendant la stérilisation des moyens 12 de filtration et de renvoyer la mesure à l'unité de contrôle.

De préférence, des moyens 70 de traitement sont interposés dans la conduite 64 d'évacuation, en amont des moyens 66 de collecte.

De tels moyens 70 de traitement comportent par exemple de l'eau et sont en particulier destinés à traiter le peroxyde d'hydrogène présent à l'état gazeux dans les effluents circulant dans la conduite 64 d'évacuation.

Des moyens 72 de mesure de la concentration en peroxyde d'hydrogène sont avantageusement associés aux moyens 70 de traitement afin de réaliser un contrôle, notamment avant évacuation vers les moyens 66 de collecte.

Les moyens 72 de mesure de la concentration en peroxyde d'hydrogène sont avantageusement utilisés pour contrôler, en fin de cycle de stérilisation, que la quantité de peroxyde d'hydrogène mesurée est inférieure à une valeur de seuil signifiant que l'ensemble du peroxyde d'hydrogène a bien été évaporé.

Le deuxième circuit C2 du dispositif 10 de stérilisation est destiné à délivrer le peroxyde d'hydrogène utilisé pour stériliser les moyens 12 de filtration.

La conduite 52 d'injection du peroxyde d'hydrogène reliée, via le raccord 48 et les moyens 50 d'injection, au premier circuit C1 d'air chaud du dispositif 10 de stérilisation comporte des moyens 74 de régulation.

Les moyens 74 de régulation, tels qu'une électrovanne, sont agencés dans la conduite 52 d'injection et sont commandés sélectivement par l'unité de contrôle (non représentée) entre au moins une position d'ouverture autorisant la circulation dans la conduite 52 et une position de fermeture interrompant la circulation dans la conduite 52.

On entend par l'expression « quantité déterminée » de peroxyde d'hydrogène, la quantité de peroxyde d'hydrogène qui est nécessaire pour stériliser les moyens de filtration pour un cycle de stérilisation.

Par ailleurs, l'expression « dose donnée» et « dose déterminée » doit s'entendre comme étant une fraction de la quantité déterminée de peroxyde d'hydrogène nécessaire pour la réalisation d'un cycle de stérilisation de filtre.

Dès lors, la somme des doses donnée correspond à la quantité déterminée de peroxyde d'hydrogène nécessaire pour réaliser un cycle de stérilisation de moyen de filtration.

Le deuxième circuit C2 comporte au moins un réservoir 76 présentant une capacité déterminée. Le réservoir 76 est destiné à être rempli avec une quantité déterminée de peroxyde d'hydrogène à l'état liquide.

De préférence, le réservoir 76 est raccordé par une conduite 78 d'alimentation à une source 80 d'alimentation en peroxyde d'hydrogène. Le peroxyde d'hydrogène présente par exemple une concentration de l'ordre de 25%.

De préférence, la conduite 78 comporte au moins une vanne 79 pour pouvoir isoler la conduite 78 et donc le deuxième circuit C2 de la source 80 de manière à permettre notamment des interventions de maintenance.

Avantageusement, la conduite 78 comporte des moyens 82 de filtration pour filtrer le peroxyde d'hydrogène en amont du réservoir 76.

Le deuxième circuit C2 comporte des moyens 84 de régulation qui sont agencés dans la conduite 78 d'alimentation pour contrôler la circulation du peroxyde d'hydrogène dans ladite conduite 78, entre la source 80 et le réservoir 76.

De préférence, les moyens 84 de régulation sont disposés en aval des moyens 82 de filtration et en amont du réservoir 76, plus précisément en amont du raccordement de la conduite 52 d'injection avec la conduite 78.

Les moyens 84 de régulation sont par exemple formés par une électrovanne, telle qu'une électrovanne de type 2/2.

Les moyens 84 de régulation sont commandés sélectivement par l'unité de contrôle (non représentée) entre au moins une position d'ouverture autorisant la circulation dans la conduite 78 et une position de fermeture interrompant la circulation dans la conduite 78.

Les moyens 84 de régulation occupant la position d'ouverture (ainsi que la vanne 79), le peroxyde d'hydrogène à l'état liquide s'écoule depuis la source 80 jusqu'au réservoir 76 en étant avantageusement filtré par les moyens 82 de filtration.

Le réservoir 76 présente une capacité déterminée correspondant par exemple à la quantité de peroxyde d'hydrogène nécessaire pour effectuer un cycle de stérilisation permettant de stériliser les moyens 12 de filtration.

Bien entendu, la capacité du réservoir 76 peut varier en fonction des applications mais pourrait également être plus importante et ne pas correspondre à la quantité nécessaire à la mise en œuvre d'un seul cycle de stérilisation.

Le deuxième circuit C2 comporte des moyens 86, 88 de mesure de niveau, tels que des sondes, pour contrôler le remplissage du réservoir 76 ou encore la purge.

Les moyens de mesure de niveau comportent au moins des premiers moyens 86 de mesure pour mesurer un niveau bas dans le réservoir 76 et des deuxièmes moyens 88 de mesure pour mesurer un niveau haut dans le réservoir 76.

Avantageusement, lesdits moyens 86 et 88 de mesure sont reliés à l'unité de contrôle (non représentée) pour commander notamment la fermeture des moyens 84 de régulation afin d'interrompre le remplissage lorsque le réservoir 76 contient la quantité désirée de peroxyde d'hydrogène.

Avantageusement, le réservoir 76 est susceptible d'être mis sous pression par l'intermédiaire d'une conduite 90 dont une extrémité est raccordée en amont au premier circuit C1 d'air comprimé et dont l'autre extrémité en aval est raccordée au réservoir 76.

De préférence, la conduite 90 se raccorde à la conduite 24 du premier circuit C1 en aval des moyens 30 de régulation de pression et en amont des moyens 32 de régulation.

Des moyens 92 de régulation sont agencés dans la conduite 90 pour contrôler la mise en pression du réservoir 76.

Les moyens 92 de régulation sont par exemple constitués par une électrovanne, telle qu'une électrovanne de type 3/2.

Les moyens 92 de régulation sont commandés sélectivement par l'unité de contrôle (non représentée) entre au moins une position d'ouverture autorisant la circulation dans la conduite 90 et une position de fermeture interrompant la circulation dans la conduite 90.

En position d'ouverture des moyens 92 de régulation, de l'air comprimé issu du premier circuit C1 est admis à l'intérieur du réservoir 76 de manière à exercer sur le peroxyde d'hydrogène liquide présent dans le réservoir 76 une pression, par exemple de l'ordre de 7 bars, soit une pression supérieure à la pression atmosphérique.

La mise sous pression assure un bon écoulement du peroxyde d'hydrogène hors du réservoir 76, en particulier vers la conduite 52 d'injection.

Les moyens 92 de régulation assurent une fonction d'évent permettant de réaliser une mise à l'air libre notamment lors du remplissage du réservoir 76 en agent stérilisant, la mise sous pression du réservoir 76 étant réalisée après son remplissage.

De préférence, le deuxième circuit C2 de peroxyde d'hydrogène comporte des moyens 94 de mesure de pression associés au réservoir 76 pour mesurer notamment la pression à l'intérieur dudit réservoir 76 et ses variations.

Les moyens 94 de mesure de pression comportent par exemple au moins un capteur de pression, relié à l'unité de contrôle.

Avantageusement, les moyens 94 de mesure de pression permettent de surveiller la variation de pression pour déterminer la quantité de peroxyde d'hydrogène injectée par l'intermédiaire de la conduite 52 d'injection.

Le deuxième circuit C2 du dispositif 10 de stérilisation comporte une conduite 96 de purge dont une extrémité amont est raccordée à la partie inférieure du réservoir 76 et dont l'autre extrémité aval est raccordée à des moyens 97 d'évacuation hors du dispositif 10 de stérilisation.

Les moyens 97 d'évacuation sont destinés à recueillir l'agent stérilisant constitué par le peroxyde d'hydrogène à l'état liquide.

De préférence, la conduite 96 de purge est raccordée à la partie inférieure du réservoir 76.

Des moyens 98 de régulation sont disposés dans la conduite 96 de purge et sont commandés pour établir sélectivement une circulation de peroxyde d'hydrogène vers les moyens 97 d'évacuation.

De préférence, les moyens 98 de régulation sont formés par au moins une électrovanne. Les moyens 98 de régulation sont commandés sélectivement par ladite unité de contrôle (non représentée) entre au moins une position d'ouverture et une position de fermeture.

En position d'ouverture, les moyens 98 de régulation autorisent une circulation de peroxyde d'hydrogène dans la conduite 96 de purge, du réservoir 76 vers les moyens 97 d'évacuation.

En position de fermeture, les moyens 98 de régulation interrompent la circulation de peroxyde d'hydrogène dans la conduite 96 de purge.

Le deuxième circuit C2 comporte une conduite 100 qui est raccordée à la conduite 78 d'alimentation, avantageusement en aval des moyens 82 de filtration.

La conduite 100 est destinée à alimenter en peroxyde d'hydrogène à l'état liquide au moins un autre dispositif de stérilisation utilisé dans l'installation de fabrication de récipients comportant le dispositif 10 de stérilisation.

De préférence, la conduite 100 comporte au moins une vanne 101 pour pouvoir interrompre la circulation de peroxyde d'hydrogène dans la conduite 100.

Avantageusement, ledit autre dispositif de stérilisation est celui utilisé pour stériliser au moins l'intérieur des préformes pour la fabrication des récipients en matière thermoplastique.

On décrira maintenant l'utilisation du dispositif 10 de stérilisation selon l'exemple de réalisation qui vient d'être décrit et plus particulièrement la mise en œuvre du procédé de stérilisation selon l'invention au moyen d'un tel dispositif 10 de stérilisation.

Tel qu'indiqué précédemment, pour procéder à la stérilisation des moyens 12 de filtration de l'air de soufflage, l'installation ou à tout le moins la machine de moulage (ou « souffleuse ») est commandée pour interrompre la fabrication de récipients et changer de mode de fonctionnement en passant du mode de fonctionnement de fabrication de récipients au mode de fonctionnement d'intervention.

Le dispositif 10 de stérilisation est également commandé sélectivement pour passer de l'état de veille à l'état d'utilisation afin de procéder à la stérilisation des moyens 12 de filtration.

Le dispositif 10 de stérilisation est en effet à l'état de veille lorsque l'installation est en mode de fonctionnement de fabrication de récipients.

Les moyens 20, 22 et 44 de régulation sont alors respectivement en position d'ouverture et les autres moyens de régulation du dispositif 10 de stérilisation sont en position de fermeture.

De préférence, le procédé de stérilisation des moyens 12 de filtration comporte une phase préliminaire qui, débutant après le changement d'état du dispositif 10 de stérilisation, consiste à réaliser une ou plusieurs étapes préparatoires avant de débuter la stérilisation des moyens 12 de filtration.

La phase préliminaire comporte au moins une étape d'isolement des moyens 12 de filtration afin notamment de pouvoir les stériliser.

L'étape d'isolement permet d'isoler les moyens 12 de filtration d'air à stériliser, en particulier par rapport au circuit 14 de soufflage d'air situé en aval desdits moyens 12 de filtration.

Avantageusement, la stérilisation est réalisée « en ligne » sans démontage des moyens 12 de filtration et automatiquement grâce au dispositif 10 de stérilisation.

L'étape d'isolement consiste à commander certains des moyens 20, 22, 44 de régulation du dispositif 10 de stérilisation, lorsqu'ils sont constitués par des électrovannes, de leur position d'ouverture vers leur position de fermeture pour isoler lesdits moyens 12 de filtration.

Pour pouvoir obtenir, d'une part, l'air chaud utilisé dans le procédé de stérilisation et, d'autre part, la vapeur de peroxyde d'hydrogène, les moyens 36 de chauffage de l'air comportant au moins ledit réchauffeur et les moyens 56 de chauffage de l'évaporateur 46 sont respectivement alimentés électriquement, mis en tension.

L'air comprimé délivré par la source 26 est introduit dans le premier circuit C1 d'air.

Avantageusement, l'air subit une ou des étapes de traitement avant d'être chauffé notamment par lesdits moyens 36 de chauffage du premier circuit C1 d'air.

L'air est chauffé par lesdits moyens 36 de chauffage durant la phase préliminaire jusqu'à atteindre une température souhaitée mais l'est également au cours de la stérilisation des moyens 12 de filtration selon le procédé.

De préférence, le procédé comporte au moins une étape de filtration de l'air consistant à filtrer l'air délivré par la source 26 d'alimentation en air comprimé.

Dans le dispositif 10 de stérilisation, l'air comprimé fournit par la source 26 d'alimentation, telle qu'un réseau de distribution d'air comprimé basse pression délivrant un air à une pression de l'ordre de 7 bars, est filtré par les moyens 28 de filtration.

De préférence, le procédé comporte au moins une étape de régulation de pression consistant à réguler en pression l'air délivré par ladite source 26 d'alimentation en air comprimé pour obtenir une pression déterminée dans le premier circuit C1 à partir de ladite source 26, de manière à avoir un débit d'air constant.

De préférence, ladite étape de régulation de pression est réalisée après l'étape de filtration de l'air comprimé grâce aux moyens 30 de régulation de pression du premier circuit C1 agencé en aval des moyens 28 de filtration.

Avantageusement, le procédé comporte au moins une étape de mesure de la pression de l'air consistant à mesurer la pression de l'air en aval desdits moyens 30 de régulation de pression afin de contrôler que la pression de l'air est égale à ladite pression déterminée.

De préférence, la phase préliminaire du procédé comporte une étape de préchauffage de l'air consistant à chauffer l'air délivré par la source 26 d'alimentation en air comprimé jusqu'à atteindre au moins une température (Tc) de consigne déterminée.

Tel qu'indiqué précédemment, l'air destiné à être chauffé est avantageusement préalablement filtré et/ou régulé en pression.

L'étape de préchauffage de l'air consiste également à chauffer l'air compris dans la cavité 54 de l'évaporateur 46 jusqu'à atteindre au moins une température (Tc) de consigne déterminée.

La température de l'air est avantageusement mesurée par les moyens 38 de mesure et par les moyens 58 de mesure pour déterminer si ladite température de l'air est au moins égale à ladite température (Tc) de consigne déterminée.

De préférence, la température (Tc) de consigne déterminée est identique pour les moyens 36 de chauffage et les moyens 56 de chauffage.

A titre d'exemple non limitatif, la valeur de la température (Tc) de consigne est d'environ 220°C.

Le procédé comporte au moins une étape de contrôle de la température de l'air réalisée au moins pendant ladite phase de préchauffage. L'étape de contrôle de la température de l'air se poursuit avantageusement ultérieurement tout au long du procédé de stérilisation.

L'étape de contrôle de la température de l'air consiste au moins à mesurer la température de l'air chauffé pour contrôler que la température de l'air est au moins égale à ladite température (Tc) de consigne.

Dans un dispositif 10 de stérilisation selon l'exemple de réalisation, ladite étape de contrôle de la température de l'air consiste à en mesurer la température par l'intermédiaire des moyens 38 de mesure de température associés aux moyens 36 de chauffage et/ou des moyens 58 de mesure de température associés auxdits moyens 56 de chauffage dudit au moins un évaporateur 46.

Lors de l'étape de préchauffage, les moyens 32, 62 et 68 de régulation du dispositif 10 de stérilisation sont en position d'ouverture et les moyens 44 de régulation en position de fermeture.

Le procédé comporte une étape de circulation d'air chaud dans le premier circuit C1 notamment pour éviter la condensation dans le premier circuit C1, en amont des moyens 12 de filtration.

L'étape de circulation d'air chaud dans le premier circuit C1 permet d'atteindre plus rapidement les valeurs de consigne de température, en particulier ladite température (Tc) de consigne.

De préférence, une circulation d'air chaud présentant une température au moins égale à ladite température (Tc) de consigne est maintenue pendant une durée déterminée, par exemple une dizaine de minutes, à travers le premier circuit C1 et les moyens 12 de filtration.

Tel qu'indiqué précédemment, le chauffage de l'air se poursuit après l'étape de préchauffage suivant une étape de chauffe consistant à chauffer en permanence, à ladite température (Tc) de consigne, l'air destiné à être utilisé pour la stérilisation.

Les moyens 36 de chauffage comportant ledit au moins un réchauffeur sont alimentés électriquement pour obtenir ledit air chaud et lesdits moyens 56 de chauffage dudit au moins un évaporateur 46 le sont également pour obtenir la vapeur de peroxyde d'hydrogène.

La phase préliminaire du procédé de stérilisation comporte également des étapes de préparation du deuxième circuit C2 de peroxyde d'hydrogène (H₂O₂).

De préférence, la phase préliminaire comporte au moins une étape de vidange consistant à purger tout ou partie du deuxième circuit C2 et notamment le réservoir 76.

L'étape de vidange consiste au moins à commander l'ouverture des moyens 92 et 98 de régulation pour purger le réservoir 76.

L'ouverture des moyens 98 de régulation autorise la circulation de peroxyde d'hydrogène à travers la conduite 96 de purge, depuis le réservoir 76 vers les moyens 97 d'évacuation.

L'ouverture des moyens 92 de régulation permet la mise à l'air libre (à la pression atmosphérique) du réservoir 76, le peroxyde d'hydrogène éventuellement présent dans le deuxième circuit C2 et tout particulièrement dans le fond du réservoir 76 est alors susceptible d'être évacué par la conduite 96 de purge.

De préférence, l'étape de vidange est interrompue après une durée déterminée, par exemple une durée d'au moins cinq secondes après l'absence de mesure (ou perte d'information) par les moyens 86 de mesure de niveau bas qui, associés au réservoir 76, sont reliés à l'unité de contrôle.

L'étape de vidange comporte, outre une purge du réservoir 76, une purge de la conduite 78 d'alimentation en peroxyde d'hydrogène du deuxième circuit C2.

Les moyens 84 de régulation sont commandés en position d'ouverture pour autoriser l'écoulement du peroxyde d'hydrogène présent dans la conduite 78 d'alimentation vers le réservoir 76, puis les moyens 98 de régulation étant également en position d'ouverture vers les moyens 97 d'évacuation.

Les moyens 92 et 98 de régulation sont maintenus en position d'ouverture lors de la purge de la conduite 78 d'alimentation.

Avantageusement, le peroxyde d'hydrogène présent dans le deuxième circuit C2 est totalement purgé grâce à l'étape de vidange afin de pouvoir garantir que les propriétés de l'agent stérilisant ne sont pas altérées.

Une fois l'étape de vidange réalisée, on procède au remplissage du réservoir 76 du deuxième circuit C2 avec du peroxyde d'hydrogène « frais » afin que le deuxième circuit C2 soit opérationnel pour délivrer ledit peroxyde d'hydrogène à l'état liquide.

L'étape de remplissage comme l'étape de vidange sont mises en œuvre lors de ladite phase préliminaire à la stérilisation des moyens 12 de filtration.

Le procédé comporte une étape de remplissage consistant à remplir ledit au moins un réservoir 76 en agent stérilisant constitué par le peroxyde d'hydrogène à l'état liquide.

Le réservoir 76 constitue des moyens de stockage du peroxyde d'hydrogène dont la capacité permet, de préférence, le stockage d'une quantité déterminée correspondant à la quantité nécessaire pour effectuer un cycle de stérilisation des moyens 12 de filtration.

Pour procéder au remplissage, on commande la fermeture des moyens 98 de régulation agencés dans la conduite 96 de purge.

Dans l'exemple de réalisation du dispositif 10 de stérilisation, l'unité de contrôle commande l'électrovanne constituant les moyens 98 de régulation de leur position d'ouverture vers leur position de fermeture.

Le remplissage du réservoir 76 s'effectue depuis la source 80 d'alimentation en peroxyde d'hydrogène à travers la conduite 78 d'alimentation.

Lors du remplissage, les moyens 84 et 92 de régulation sont en position d'ouverture et les moyens 74 de régulation en position de fermeture.

Le remplissage se poursuit jusqu'à ce que les moyens 88 de mesure de niveau haut, associés au réservoir 76, détectent du peroxyde d'hydrogène.

A la fin de l'étape de remplissage, le réservoir 76 contient au moins la quantité déterminée de peroxyde d'hydrogène liquide pour stériliser lesdits moyens 12 de filtration selon le procédé.

La phase préliminaire est alors achevée et la phase de stérilisation proprement dite des moyens 12 de filtration peut débuter.

Le procédé de stérilisation des moyens 12 de filtration de gaz comporte au moins :
une phase préliminaire comportant :
   - une étape d'isolement des moyens 12 de filtrations, en particulier par rapport au circuit de soufflage d'air situé en aval desdits moyens de filtration à stériliser;
   - une étape de préchauffage de l'air utilisé dans le procédé de stérilisation consistant à chauffer l'air délivré par la source d'alimentation en air comprimé jusqu'à atteindre au moins une température Tc de consigne déterminée;
   Quand la phase préliminaire est achevée alors une phase de stérilisation peut débuter ;
la phase de stérilisation comporte :
   - une étape d'application consistant à faire circuler à travers les moyens 12 de filtration de gaz un mélange gazeux comportant de l'air chaud et une quantité déterminée de vapeur de peroxyde d'hydrogène, dans lequel ladite quantité déterminée de vapeur de peroxyde d'hydrogène est obtenue en injectant séquentiellement, avec un intervalle (t) de temps donné entre deux injections successives, une dose donnée de peroxyde d'hydrogène à l'état liquide dans l'air chaud; et
   - une étape de stérilisation consistant, pendant ledit intervalle (t) de temps, à maintenir la circulation de l'air chaud à travers lesdits moyens de filtration pour éliminer par évaporation tout ou partie du peroxyde d'hydrogène déposé sur lesdits moyens de filtration lors de ladite étape d'application.
Dans ce procédé de stérilisation de moyen de filtration, le même air chaud est utilisé dans l'étape d'application comme dans l'étape de stérilisation.

En d'autres termes, après avoir réalisé la phase préliminaire pour la mise en œuvre du procédé de stérilisation de filtre, ledit procédé comprend une application continue d'un flux d'air chaud traversant au moins un filtre à stériliser, une étape d'injection d'une dose donnée de peroxyde d'hydrogène dans le flux d'air chaud pour appliquer le peroxyde d'hydrogène sur le filtre, cette étape d'application est suivit d'une étape de stérilisation consistant à laisser du temps au flux d'air chaud pour évaporer le peroxyde d'hydrogène injecté lors de la précédente étape et se trouvant au niveau du filtre, ces deux étapes sont répétées au tant de fois qu'il est nécessaire pour que la quantité déterminée de peroxyde d'hydrogène soit atteinte pour la réalisation du cycle de stérilisation du moyen de filtration.

Le procédé de stérilisation des moyens 12 de filtration de gaz comporte au moins :
- une étape d'application consistant à faire circuler, à travers les moyens 12 de filtration de gaz, un mélange gazeux comportant de l'air chaud et une quantité déterminée de vapeur de peroxyde d'hydrogène, dans lequel ladite quantité déterminée de vapeur de peroxyde d'hydrogène est obtenue en injectant séquentiellement, avec un intervalle (t) de temps donné entre deux injections successives, une dose donnée de peroxyde d'hydrogène à l'état liquide dans l'air chaud; et
- une étape de stérilisation consistant, pendant au moins ledit intervalle (t) de temps, à faire circuler de l'air chaud à travers lesdits moyens 12 de filtration pour éliminer par évaporation tout ou partie du peroxyde d'hydrogène déposé sur lesdits moyens 12 de filtration lors de ladite étape d'application.

Le procédé de stérilisation consiste à réaliser tout d'abord une étape d'application d'un mélange gazeux contenant du peroxyde d'hydrogène à l'état de vapeur sur les moyens 12 de filtration.

L'application de l'agent stérilisant formé par le peroxyde d'hydrogène s'effectue en faisant circuler, à travers les moyens 12 de filtration, un mélange gazeux constitué d'air chaud et d'une fraction de la quantité déterminée de vapeur de peroxyde d'hydrogène.

Selon une caractéristique importante le mélange gazeux comporte une fraction de la quantité déterminée de vapeur de peroxyde d'hydrogène résultant de l'injection séquentielle dans l'air chaud d'une dose donnée de peroxyde d'hydrogène à l'état liquide.

L'injection du peroxyde d'hydrogène à l'état liquide est réalisée séquentiellement avec un intervalle (t) de temps donné entre deux injections successives.

Pour obtenir ledit mélange gazeux, le procédé comporte au moins une étape de vaporisation du peroxyde d'hydrogène.

L'étape de vaporisation consiste à vaporiser ladite dose donnée de peroxyde d'hydrogène à l'état liquide dans des moyens d'évaporation formés par l'évaporateur 46 pour obtenir ledit mélange gazeux utilisé lors de l'étape d'application.

Avantageusement, la vaporisation est obtenue en injectant ladite dose donnée de peroxyde d'hydrogène à l'état liquide dans un flux continu (ou « veine ») d'air chaud puis à introduire l'ensemble dans l'évaporateur 46 pour obtenir ledit mélange gazeux utilisé lors de l'étape d'application.

Avec un dispositif 10 de stérilisation selon l'exemple de réalisation, l'injection de ladite dose donnée de peroxyde d'hydrogène au cours de l'étape de vaporisation est obtenue en commandant sélectivement en ouverture les moyens 74 de régulation pendant un laps de temps donné.

L'ouverture des moyens 74 de régulation pendant ledit laps de temps donné permet, pour un débit de peroxyde d'hydrogène déterminé dans la conduite 52 d'injection, d'injecter par l'intermédiaire des moyens 50 d'injection ladite dose donnée voulue dans l'air chaud circulant en permanence à travers le raccord 48.

Dans le dispositif 10 de stérilisation, l'injection séquentielle est obtenue en commandant sélectivement en fermeture lesdits moyens 74 de régulation pendant ledit intervalle (t) de temps donné entre deux injections successives d'une dose donnée de peroxyde d'hydrogène à l'état liquide.

A titre d'exemple non limitatif sur une séquence d'une durée totale de trente secondes, les moyens 74 de régulation sont commandés en position d'ouverture pendant un laps de temps d'une seconde pour injecter ladite dose donnée dans l'air chaud, puis sont commandés en position de fermeture pendant un intervalle de temps de vingt-neuf secondes.

Au cours dudit intervalle de temps (t), aucun peroxyde d'hydrogène n'est plus injecté par les moyens 50 d'injection et seul l'air chaud délivré par la conduite 24 du premier circuit C1 continue de circuler, depuis l'évaporateur 46, à travers la conduite 60 et les moyens 12 de filtration.

C'est au moins pendant cet intervalle (t) de temps qu'est réalisée ladite étape de stérilisation. En effet, l'étape de stérilisation consiste à faire circuler de l'air chaud à travers lesdits moyens 12 de filtration pour éliminer par évaporation tout ou partie du peroxyde d'hydrogène préalablement déposé lors de ladite étape d'application.

Avantageusement, le peroxyde d'hydrogène est déposé par condensation sur les moyens 12 de filtration lors de ladite étape d'application.

Par comparaison avec une injection continue, l'injection séquentielle d'une dose donnée de peroxyde d'hydrogène permet de déposer une fraction de la quantité déterminée de peroxyde d'hydrogène sur les moyens 12 de filtration.

Ladite dose donnée de peroxyde d'hydrogène est notamment déterminée de manière à ne pas saturer, colmater les moyens 12 de filtration.

Grâce à l'injection séquentielle, la fraction de ladite quantité déterminée de vapeur de peroxyde d'hydrogène qui se condense sur les moyens 12 filtration est susceptible d'être activée thermiquement et évaporée par l'air chaud pendant ledit intervalle (t) de temps.

Dans un dispositif 10 de filtration, la dose donnée de peroxyde d'hydrogène à l'état liquide est introduite par l'air chaud dans la cavité 54 de l'évaporateur 46 où le peroxyde d'hydrogène est alors vaporisé par les moyens 56 de chauffage.

Le mélange gazeux en résultant est constitué d'air chaud et d'une fraction de la quantité déterminée de vapeur de peroxyde d'hydrogène qui est ensuite acheminée par la conduite 60 jusqu'aux moyens 12 de filtration à stériliser.

Les moyens 62 de régulation sont en position d'ouverture autorisant la circulation dudit mélange gazeux depuis l'évaporateur 46 jusqu'aux moyens 12 de filtration.

La partie du mélange gazeux qui traverse lesdits moyens 12 de filtration est ensuite évacuée par l'intermédiaire de la conduite 64 d'évacuation en direction des moyens 66 de collecte, les moyens 68 de régulation étant en position d'ouverture.

En fonction de la valeur de l'intervalle (t) de temps, l'étape de stérilisation qui succède à l'étape d'application permet d'éliminer tout ou partie des condensats de peroxyde d'hydrogène avec l'air chaud et ceci avant de procéder à une nouvelle étape d'application par injection de la dose donnée suivante.

Lorsque la valeur de l'intervalle (t) de temps est trop courte pour garantir notamment une élimination complète du peroxyde d'hydrogène, le procédé de stérilisation comporte alors avantageusement une étape de stérilisation, dite complémentaire, consistant à faire circuler uniquement de l'air chaud à travers lesdits moyens 12 de filtration pendant une durée (D) déterminée.

L'étape de stérilisation complémentaire est réalisée une fois achevée l'application, sous la forme desdites doses successivement injectées, de la quantité souhaitée de peroxyde d'hydrogène, laquelle quantité correspond par exemple à la capacité du réservoir 76.

A titre d'exemple non limitatif, l'étape de stérilisation complémentaire consiste à faire circuler à travers les moyens 12 de filtration uniquement de l'air chaud pendant une durée D entre quinze à quarante minutes, de préférence de vingt-cinq minutes pour garantir une évaporation complète du peroxyde d'hydrogène.

Au moins une partie de la stérilisation « chimique » des moyens 12 de filtration selon le procédé se produit donc pendant l'intervalle (t) de temps et peut au besoin s'achever lors d'une telle étape de stérilisation complémentaire.

Lorsque le mélange gazeux amené par la conduite 60 entre en contact avec les moyens 12 de filtration dont la température est inférieure, le peroxyde d'hydrogène à l'état de vapeur va alors se déposer par condensation sur les moyens 12 de filtration.

A l'issue de l'étape d'application, les moyens 12 de filtration comportent au moins en surface des condensats de peroxyde d'hydrogène résultant du changement d'état, de l'état de vapeur à l'état liquide, survenu lors de l'entrée en contact de la vapeur de peroxyde d'hydrogène contenue dans le mélange gazeux avec lesdits moyens 12 de filtration.

Une fois l'injection de ladite dose donnée de peroxyde d'hydrogène et celle-ci vaporisée dans l'évaporateur 46, la conduite 24 continue de délivrer de l'air chaud à la température (Tc) de consigne.

Cet air chaud traverse l'évaporateur 46 et poursuit son chemin dans le dispositif 10 de stérilisation jusqu'à venir au contact des moyens 12 de filtration.

L'air chaud entrant en contact avec les moyens 12 de filtration présente alors par exemple une température comprise entre 100°C et 120°C qui va provoquer progressivement l'évaporation desdits condensats de peroxyde d'hydrogène.

L'air chaud active thermiquement le peroxyde d'hydrogène (H₂O₂) en agissant sur les liaisons chimiques du peroxyde d'hydrogène, en cassant lesdites liaisons l'air chaud provoque l'apparition de radicaux libres (OH) très actifs qui vont détruire les micro-organismes et permettre d'obtenir la stérilisation recherchée.

L'air chaud évapore progressivement l'eau présente dans les condensats de peroxyde d'hydrogène ce qui provoque au fur et à mesure une augmentation de la concentration en peroxyde d'hydrogène et ce faisant accroit l'effet stérilisant.

Une fois évaporé, le peroxyde d'hydrogène à l'état gazeux traverse aisément les moyens 12 de filtration et cela sans en altérer la structure.

Avantageusement, le procédé comporte une étape de traitement consistant à traiter les effluents issus de la stérilisation et tout particulièrement le peroxyde d'hydrogène à l'état gazeux.

C'est la raison pour laquelle, le dispositif 10 de stérilisation comporte des moyens 70 de traitement agencés dans la conduite 64 d'évacuation, en amont des moyens 66 de collecte.

Un cycle de stérilisation selon le procédé consiste à répéter successivement un nombre « n » de fois la séquence constituée desdites étapes d'application et de stérilisation.

Lors d'une séquence du cycle de stérilisation, les moyens 74 de régulation sont commandés en ouverture pour injecter ladite dose déterminée de peroxyde d'hydrogène, dans l'exemple par l'intermédiaire des moyens 50 d'injection.

L'étape d'application est alors effectuée telle que cela a été décrit précédemment.

Les moyens 74 de régulation sont ensuite commandés en fermeture pendant l'intervalle (t) de temps afin de réaliser l'étape de stérilisation.

Suivant les exemples de valeurs indiquées précédemment, pour une séquence d'une durée totale de trente secondes, les moyens 74 de régulation sont ouverts pendant une seconde puis fermés pendant vingt-neuf secondes.

Lorsque l'intervalle (t) de temps est relativement court comme dans l'exemple ci-dessus, une étape de stérilisation complémentaire est alors effectuée par exemple pendant une durée D comprise entre quinze à quarante minutes, de préférence de vingt-cinq minutes.

Ainsi qu'on l'aura compris ladite étape de stérilisation complémentaire est toutefois facultative et pourrait avantageusement être supprimée en augmentant ledit intervalle (t) de temps, par exemple à environ cinq minutes entre chaque injection de peroxyde d'hydrogène.

Avantageusement, la séquence desdites étapes d'application et de stérilisation est répétée un nombre « n » de fois, par exemple entre cinq et vingt-cinq fois selon les applications.

La répétition de la séquence est indépendante de la valeur de l'intervalle (t) de temps et de la mise en œuvre de ladite étape de stérilisation complémentaire.

De préférence, la capacité du réservoir 76 correspond à la quantité déterminé de peroxyde d'hydrogène nécessaire à la répétition de la séquence, soit à au moins à « n » fois ladite dose donnée de peroxyde d'hydrogène injectée séquentiellement.

Pour un cycle de stérilisation, on effectue alors initialement qu'une seule étape de remplissage lors de la phase préliminaire de préparation du dispositif 10 de stérilisation.

Tel qu'expliqué précédemment, la répétition alternée desdites étapes d'application d'une fraction de la quantité déterminée de vapeur de peroxyde d'hydrogène issue de ladite dose donnée et d'air chaud permet avantageusement de ne pas saturer les moyens 12 de filtration.

Pour une quantité égale de vapeur de peroxyde d'hydrogène, la stérilisation des moyens 12 de filtration obtenue est ainsi meilleure en effectuant successivement « n » fois la séquence formée desdites étapes d'application et de stérilisation, qu'en projetant en une seule fois une quantité équivalente de vapeur de peroxyde d'hydrogène.

Avec l'injection séquentielle selon le procédé de stérilisation de l'invention, l'élimination du peroxyde d'hydrogène par évaporation au moyen de l'air chaud requiert par comparaison moins d'énergie qu'il n'en faudrait pour obtenir l'évaporation d'une quantité équivalente qui serait injectée en une seule et unique fois.

Pour évaporer une quantité équivalente de peroxyde d'hydrogène injectée non pas séquentiellement mais en une seule et unique fois, il serait en effet nécessaire d'augmenter la température de l'air chaud ce qui aurait pour conséquence d'accroître l'énergie nécessaire, voir également nécessaire d'augmenter le débit de l'air chaud.

Or une telle augmentation de la température et/ou du débit d'air chaud utilisé dans le procédé de stérilisation aurait pour conséquence de risquer de détériorer les moyens 12 de filtration comme c'était le cas auparavant avec la vapeur d'eau.

Avantageusement, le procédé de stérilisation selon l'invention ne permet donc pas seulement de stériliser efficacement les moyens 12 de filtration mais également de ne pas les détériorer pour en permettre à nouveau l'utilisation après la stérilisation.

En préservant les moyens 12 de filtration, le procédé de stérilisation selon l'invention permet avantageusement d'en augmenter la durée de vie, ce qui contribue à réduire les coûts.

Le procédé et le dispositif de stérilisation de moyens de filtration de gaz sont notamment destinés à être utilisés pour la stérilisation de moyens de filtration d'air de soufflage dans une installation de fabrication de récipients en matière thermoplastique à partir de préformes chaudes.

## Revendications

1. Procédé de stérilisation de moyens de filtration de gaz, comportant au moins :
- une étape d'application consistant à faire circuler à travers les moyens de filtration de gaz un mélange gazeux comportant de l'air chaud et une quantité déterminée de vapeur de peroxyde d'hydrogène, dans lequel ladite quantité déterminée de vapeur de peroxyde d'hydrogène est déterminée comme étant nécessaire pour stériliser les moyens de filtration de gaz pour un cycle de stérilisation; et
- une étape de stérilisation consistant, à faire circuler de l'air chaud à travers lesdits moyens de filtration pour éliminer par évaporation tout ou partie du peroxyde d'hydrogène déposé sur lesdits moyens de filtration lors de ladite étape d'application ;
durant l'étape d'application, ladite quantité déterminée de vapeur de peroxyde d'hydrogène est obtenue en injectant séquentiellement, avec un intervalle (t) de temps donné entre deux injections successives, une dose donnée de peroxyde d'hydrogène à l'état liquide dans l'air chaud et en ce que ladite dose donnée étant une fraction de la quantité déterminée et l'étape de stérilisation a lieu pendant ledit intervalle (t) de temps.

2. Procédé de stérilisation selon la revendication 1, **caractérisé en ce que** le procédé comporte au moins :
- une étape de vaporisation consistant à vaporiser ladite dose donnée de peroxyde d'hydrogène à l'état liquide dans des moyens d'évaporation pour obtenir ledit mélange gazeux utilisé lors de l'étape d'application.

3. Procédé de stérilisation selon la revendication 2, **caractérisé en ce que** le procédé comporte au moins :
- une étape d'injection consistant à injecter séquentiellement avec ledit intervalle (t) de temps donné entre deux injections successives ladite dose donnée de peroxyde d'hydrogène à l'état liquide dans un flux continu d'air chaud et à introduire l'ensemble dans lesdits moyens d'évaporation pour obtenir ledit mélange gazeux.

4. Procédé de stérilisation selon la revendication 3, **caractérisé en ce que** l'étape d'injection de ladite dose donnée de peroxyde d'hydrogène est réalisée séquentiellement en commandant sélectivement des moyens de régulation, respectivement en position d'ouverture pendant un laps de temps donné et en position de fermeture pendant un intervalle (t) de temps donné entre deux injections successives de ladite dose donnée de peroxyde d'hydrogène à l'état liquide.

5. Procédé de stérilisation selon l'une quelconque des revendications 1 à 4, **caractérisé en ce que** le procédé de stérilisation consiste à réaliser au moins un cycle au cours duquel une séquence, comportant lesdites étapes d'application et de stérilisation, est répétée un nombre « n » de fois.

6. Procédé de stérilisation selon la revendication 5, **caractérisé en ce que** le procédé comporte au moins :
- une étape de stérilisation complémentaire consistant à faire circuler uniquement de l'air chaud à travers lesdits moyens de filtration pendant une durée (D) qui est déterminée en fonction de l'intervalle (t) de temps pour garantir une évaporation du peroxyde d'hydrogène.

## Patentansprüche

1. Verfahren zur Sterilisierung von Gasfiltermitteln, umfassend mindestens:
- einen Anwendungsschritt, der darin besteht, durch die Gasfiltermittel ein Gasgemisch strömen zu lassen, das heiße Luft und eine bestimmte Menge Wasserstoffperoxiddampf umfasst, wobei die bestimmte Menge Wasserstoffperoxiddampf als zum Sterilisieren der Gasfiltermittel für einen Sterilisierungszyklus erforderlich bestimmt wird; und
- einen Sterilisierungsschritt, der darin besteht, heiße Luft durch die Filtermittel strömen zu lassen, um durch Verdampfen das gesamte oder einen Teil des Wasserstoffperoxids, das sich bei dem Anwendungsschritt auf den Filtermitteln abgesetzt hat, zu entfernen;
während des Anwendungsschritts die bestimmte Menge Wasserstoffperoxiddampf erhalten wird, indem sequenziell, mit einem gegebenen Zeitintervall (t) zwischen zwei aufeinander folgenden Einleitungen, eine gegebene Dosis Wasserstoffperoxid in flüssigem Zustand in die heiße Luft eingeleitet wird, und dadurch, dass die gegebene Dosis ein Bruchteil der bestimmten Menge ist und der Sterilisierungsschritt während des Zeitintervalls (t) stattfindet.

2. Verfahren zur Sterilisierung nach Anspruch 1, **dadurch gekennzeichnet, dass** das Verfahren mindestens umfasst:
- einen Verdampfungsschritt, der darin besteht, die gegebene Dosis Wasserstoffperoxid in flüssigem Zustand in Verdampfungsmitteln zu verdampfen, um das bei dem Anwendungsschritt verwendete Gasgemisch zu erhalten.

3. Verfahren zur Sterilisierung nach Anspruch 2, **dadurch gekennzeichnet, dass** das Verfahren mindestens umfasst:
- einen Schritt des Einleitens, der darin besteht, die gegebene Dosis Wasserstoffperoxid in flüssigem Zustand sequenziell, mit dem gegebenen Zeitintervall (t) zwischen zwei aufeinander folgenden Einleitungen, in einen kontinuierlichen Strom heißer Luft einzuleiten und das Ganze in die Verdampfungsmittel einzuführen, um das Gasgemisch zu erhalten.

4. Verfahren zur Sterilisierung nach Anspruch 3, **dadurch gekennzeichnet, dass** der Schritt des Einleitens der gegebenen Dosis Wasserstoffperoxid sequenziell ausgeführt wird, indem Regulierungsmittel selektiv in eine Öffnungsstellung während eines gegebenen Zeitraums beziehungsweise in eine Schließstellung während eines gegebenen Zeitraums während eines gegebenen Zeitintervalls (t) zwischen zwei aufeinander folgenden Einleitungen der gegebenen Dosis Wasserstoffperoxid in flüssigem Zustand gesteuert werden.

5. Verfahren zur Sterilisierung nach einem der Ansprüche 1 bis 4, **dadurch gekennzeichnet, dass** das Verfahren zur Sterilisierung darin besteht, mindestens einen Zyklus auszuführen, während dessen eine Sequenz, die die Anwendungs- und Sterilisierungsschritte umfasst, "n" Mal wiederholt wird.

6. Verfahren zur Sterilisierung nach Anspruch 5, **dadurch gekennzeichnet, dass** das Verfahren mindestens umfasst:
- einen ergänzenden Sterilisierungsschritt, der darin besteht, während einer Dauer (D), die in Abhängigkeit von dem Zeitintervall (t) bestimmt wird, nur heiße Luft durch die Filtermittel strömen zu lassen, um ein Verdampfen des Wasserstoffperoxids zu garantieren.

## Claims

1. Method for sterilizing gas filtration means, comprising at least:
- an application step consisting in circulating, through the gas filtration means, a gaseous mixture comprising hot air and a determined quantity of hydrogen peroxide vapour, wherein said determined quantity of hydrogen peroxide vapour is determined as being necessary to sterilize the gas filtration means for a sterilization cycle; and
- a sterilization step consisting in circulating hot air through said filtration means to eliminate by evaporation all or part of the hydrogen peroxide deposited on said filtration means during said application step;
during the application step, said determined quantity of hydrogen peroxide vapour is obtained by injecting sequentially, with a given time interval (t) between two successive injections, a given dose of hydrogen peroxide in the liquid state into the hot air and in that said given dose is a fraction of the determined quantity and the sterilization step takes place during said time interval (t).

2. Sterilization method according to Claim 1, **characterized in that** the method comprises at least:
- a vaporization step consisting in vaporizing said given dose of hydrogen peroxide in the liquid state in evaporation means to obtain said gaseous mixture used during the application step.

3. Sterilization method according to Claim 2, **characterized in that** the method comprises at least:
- an injection step consisting in injecting sequentially, with said given time interval (t) between two successive injections, said given dose of hydrogen peroxide in the liquid state into a continuous stream of hot air and in introducing the whole into said evaporation means to obtain said gaseous mixture.

4. Sterilization method according to Claim 3, **characterized in that** the step of injecting said given dose of hydrogen peroxide is carried out sequentially by selectively commanding regulation means, respectively into an open position for a given time period and into a closed position for a given time interval (t) between two successive injections of said given dose of hydrogen peroxide in the liquid state.

5. Sterilization method according to any one of Claims 1 to 4, **characterized in that** the sterilization method consists in carrying out at least one cycle during which a sequence, comprising said application and sterilization steps, is repeated a number "n" of times.

6. Sterilization method according to Claim 5, **characterized in that** the method comprises at least:
- an additional sterilization step consisting in circulating only hot air through said filtration means for a duration (D) that is determined as a function of the time interval (t) to ensure evaporation of the hydrogen peroxide.
